# EUROPEAN PATENT APPLICATION

(11) **EP 4 545 084 A1**
(43) Date of publication of application: **30.04.2025**
(21) Application number: 23827286.8
(22) Date of filing: 23.06.2023
(51) Int. Cl.: A61K 35/12, C12M 1/00, C12M 1/38, C12N 1/04, C12N 5/078

(54) **SYSTEM FOR PROVIDING CELLULAR PRODUCT**

(30) Priority: 24.06.2022 JP 2022101485
(71) Applicant: FUJIFILM Corporation, Tokyo 106-8620 (JP)
(72) Inventor: NAKAMURA Kentaro, Ashigarakami-gun, Kanagawa 258-8577 (JP)
(74) Representative: Hoffmann Eitle
(86) International application number: PCT/JP2023/023259
(87) International publication number: WO 2023/249104

(57) **Abstract**

An object of the present invention is to provide a system for finally providing one kind of a cell product in a state where it functions as a cell product without mixing or confusing raw materials in a case where the cell product is manufactured using two or more kinds of tissues derived from the same patient.

According to the present invention, there is provided a system for providing a cell product including: an identification step of assigning an identification code representing patient information to two or more kinds of raw materials derived from the same patient; a first transport step of transporting the two or more kinds of raw materials to which the identification code is assigned from a medical institution to a manufacturing site under a condition where a product of a temperature (°C) and the number of days (days) in a transport period is in a predetermined numerical range; a manufacturing step of manufacturing a cell product by recognizing the identification code and combining the two or more kinds of individual raw materials into one product; and a second transport step of transporting the cell product from the manufacturing site to the medical institution.

## Description

### Technical Field

The present invention relates to a system for providing a cell product manufactured using two or more kinds of raw materials derived from the same patient.

### Background Art

In recent years, with the progress of regenerative medicine technology and cell therapy technology, various kinds of cell therapy using autologous, allogeneic, or heterologous cells, and the development of a cell product for research have been actively carried out. For example, the mesenchymal stem cell (MSC) is expected as a cell source useful for cell therapy. Mesenchymal stem cells can be collected from various body tissues and can be isolated from bone marrow tissue, adipose tissue, muscle tissue, synovial tissue, periosteal tissue, and the like. In particular, it has been reported that the synovium-derived mesenchymal stem cells (also referred to as synovial stem cells) have higher proliferation ability and higher chondrogenesis ability as compared with mesenchymal stem cells derived from various mesenchymal tissues such as bone marrow. In addition, Patent Document 1 discloses a method of treating articular cartilage injury and meniscus injury by using synovium-derived mesenchymal stem cells.

In addition, Patent Document 2 describes a preservative solution for human stem cells and a method for preserving human stem cells using the above-described preservative solution. The preservative solution includes at least human serum, in which a volume ratio of human serum with respect to the entire preservative solution is 0.70 or more, and the human stem cells are preserved at a temperature higher than 0°C.

### Prior Art Documents

### Patent Documents

Patent Document 1: JP5656183B
Patent Document 2: WO2018/147457A

### SUMMARY OF THE INVENTION

### OBJECT TO BE SOLVED BY THE INVENTION

Regarding the cells for treatment, the cells themselves are not a perfect uniform constituent, it is difficult to specify characteristics of the cells, and the cells have a property of viability (that is, it is necessary to distinguish between living cells and dead cells). Therefore, it is difficult to manage the cells for treatment as a completely defined material, and thus the cells for treatment are recognized through development of a cell product. Actually, even in a manufacturing site of the cell product, the cells are not managed as a final product, and it is necessary to control the characteristics of the cell product by controlling the manufacturing process. However, actually, with the simple method of controlling the manufacturing process in the manufacturing site, it is difficult to appropriately provide the cells to treatment. Accordingly, as a system for providing a cell product, there is required a system where a process of administering a manufactured cell product to a patient after collecting a starting material from the patient is controlled.

In the related art, cells are isolated from a tissue derived from a patient in a medical institution, and the cells are provided to treatment. On the other hand, it has been assumed that two or more kinds of raw materials derived from the same patient are used, the two or more kinds of raw materials derived from the patient are transported to a manufacturing site other than a medical institution, the two or more kinds of raw materials derived from the patient are combined to manufacture one kind of a cell product in the manufacturing site, and the manufactured cell product is transported to the medical institution and administered to the patient.

The present inventors focused on a new issue that, in a case where two or more kinds of tissues derived from the same patient are used as described above, it is necessary that the quality of the two or more kinds of raw materials derived from the same patient is maintained, one kind of a cell product is finally manufactured in a state where the cells are alive without mixing or confusing the raw materials, the cell product is transported to a medical institution in a state where the cells are alive, and the cell product is provided in a state where it functions as a cell product (the cell viability in the cell product is 70% or more). That is, an object of the present invention is to provide a system for finally providing one kind of a cell product in a state where it functions as a cell product without mixing or confusing raw materials in a case where the cell product is manufactured using two or more kinds of tissues derived from the same patient.

### MEANS FOR SOLVING THE OBJECT

The present inventors conducted a thorough investigation in order to achieve the above-described object and found that the above-described object can be achieved by assigning an identification code representing patient information to two or more kinds of individual raw materials derived from the same patient, transporting the two or more kinds of individual raw material to which the identification code is assigned from a medical institution to a manufacturing site under a predetermined appropriate condition, recognizing the identification code after the transport, and combining the above-described two or more kinds of individual raw material into one product to manufacture a cell product. The present invention has been completed based on the above findings.

According to the present invention, the following inventions are provided.
<1> A system for providing a cell product, the system comprising:
   an identification step of assigning an identification code representing patient information to each of two or more kinds of individual raw materials derived from the same patient;
   a first transport step of transporting the two or more kinds of individual raw materials to which the identification code is assigned from a medical institution to a manufacturing site in a state where each of the raw materials is encapsulated in a double or more multiple packaging container, in which at least one of the two or more kinds of individual raw materials is transported from the medical institution to the manufacturing site under a condition where a product of a temperature (°C) and the number of days (days) in a transport period is 170 (°C × days) or less;
   a manufacturing step including manufacturing a cell product by recognizing the identification code and combining the two or more kinds of individual raw materials into one product after the first transport step; and
   a second transport step of transporting the cell product from the manufacturing site to the medical institution after the manufacturing step.
<2> The system according to <1>,
   in which a temperature of the first transport step is 1°C to 27°C, and the transport period is 1 hour or longer and 160 hours or shorter.
<3> The system according to <1> or <2>,
   in which the manufacturing step further includes
   a step of separating the two or more kinds of individual raw materials into respective members one of which is a cell, culturing the cell at a temperature of 30°C or higher, and subsequently determining whether or not the cell is suitable for a quality standard.
<4> The system according to any one of <1> to <3>,
   in which a biological raw material other than the raw materials derived from the patient is not used.
<5> The system according to any one of <1> to <4>,
   in which the first transport step and the second transport step include measuring and recording a temperature and tracing a record of the temperature.
<6> The system according to any one of <1> to <5>,
   in which one of the two or more kinds of individual raw materials is blood.
<7> The system according to <6>,
   in which a period of the first transport step for the blood is 150 hours or shorter.
<8> The system according to any one of <1> to <7>,
   in which one of the two or more kinds of individual raw materials is synovial tissue.
<9> The system according to <8>,
   in which a period of the first transport step for the synovial tissue is 130 hours or shorter.
<10> The system according to any one of <1> to <9>,
   in which the two or more kinds of individual raw materials include blood and synovial tissue.
<11> The system according to any one of <1> to <10>,
   in which an interval between dates of sending at which the two or more kinds of individual raw materials are sent from the medical institution, respectively, or an interval between dates of arrival at which the two or more kinds of individual raw materials arrive at the manufacturing site, respectively, is 3 days to 2 weeks.
<12> The system according to any one of <1> to <11>,
   in which in the second transport step, a transport time is 1 hour or longer and 90 hours or shorter, and a transport temperature is 30°C or lower.
<13> The system according to any one of <1> to <12>,
   in which a second identification code that is coupled to the identification code assigned to the two or more kinds of individual raw materials is assigned to the cell product, and the cell product is allocated to the patient from which the raw materials are derived based on the second identification code.

### EFFECT OF THE INVENTION

According to the present invention, it is possible to provide a system for finally providing one kind of a cell product in a state where it functions as a cell product without mixing or confusing raw materials in a case where the cell product is manufactured using two or more kinds of tissues derived from the same patient.

### BRIEF DESCRIPTION OF THE DRAWINGS

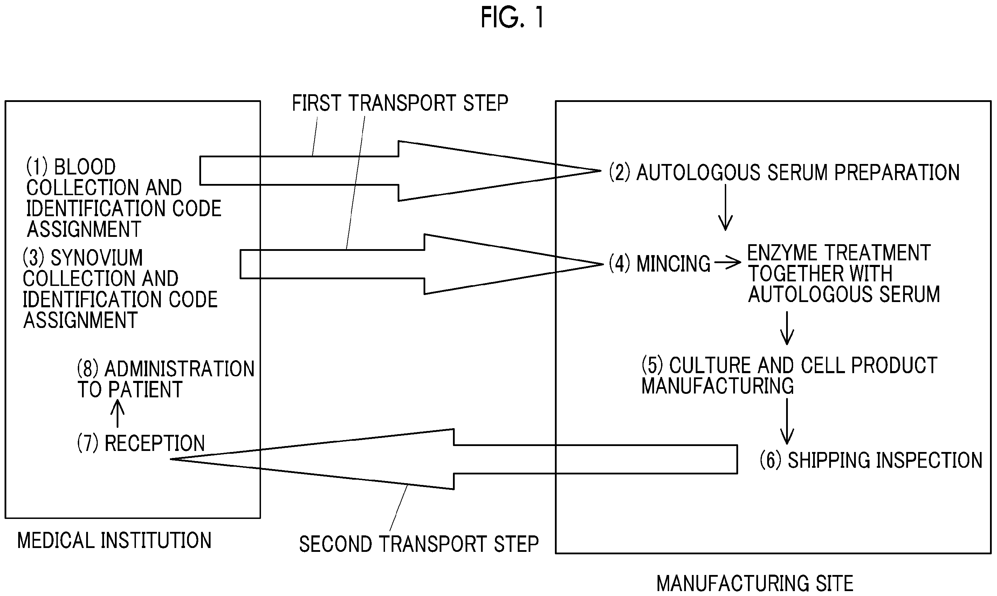

[Fig. 1] Fig. 1 is a schematic diagram showing an example of a system for providing a cell product according to the present invention.

### EMBODIMENTS FOR CARRYING OUT THE INVENTION

Hereinafter, an example of an embodiment of the present disclosure will be described. Note that the present disclosure is not limited to the following embodiment, and can be implemented with appropriate changes within the scope of the object of the present disclosure. In the present specification, a numerical range indicated using "to" represents a range including numerical values described before and after "to" as a minimum value and a maximum value, respectively.

A system for providing a cell product according to the present invention comprises:
an identification step of assigning an identification code representing patient information to each of two or more kinds of individual raw materials derived from the same patient;
a first transport step of transporting the two or more kinds of individual raw materials to which the identification code is assigned from a medical institution to a manufacturing site in a state where each of the raw materials is encapsulated in a double or more multiple packaging container, in which at least one of the two or more kinds of individual raw materials is transported from the medical institution to the manufacturing site under a condition where a product of a temperature (°C) and the number of days (days) in a transport period is 170 (°C × days) or less;
a manufacturing step including manufacturing a cell product by recognizing the identification code and combining the two or more kinds of individual raw materials into one product after the first transport step; and
a second transport step of transporting the cell product from the manufacturing site to the medical institution after the manufacturing step.

Cell therapy is a treatment (including prevention and relaxation) for disease or injury of a target (for example, mammal, preferably, human) by administering autologous, allogeneic, or heterologous cells that are processed outside the body. The cell product refers to a product used in the cell therapy (including prevention and relaxation), which is a medicine including at least the cells. By administering the living cells to the target from the outside of the body to exert the cell function in the body, the disease in the target can be treated or prevented. The cell product according to the present invention is a product manufactured by combining two or more kinds of raw materials derived from the same patient.

In the system according to the present invention, the two or more kinds of raw materials derived from the patient are used. It is preferable that a biological raw material other than the above-described raw materials derived from the patient is not used.

The system according to the present invention may be a method for providing a cell product including the identification step, the first transport step, the manufacturing step, and the second transport step described above, or may be means for performing the above-described method for providing a cell product.

### <Identification Step>

The identification step is a step of assigning an identification code representing patient information to each of two or more kinds of individual raw materials derived from the same patient. The identification step can be performed in the medical institution, for example, a hospital, a clinic, or a medical office.

In the present invention, the two or more kinds of raw materials derived from the same patient are used. As long as the number of the kinds of the raw materials is two or more, three or more kinds of raw materials may also be used. The upper limit of the number of the kinds of the raw materials is not particularly limited and is generally 5 or less.

As the raw materials derived from the patient, tissues or organs of the patient can be used, and examples thereof include synovium, blood (for example, peripheral blood), bone marrow, periosteum, synovium, adipose tissue, muscle, pulp, placental tissue, umbilical tissue (umbilical blood), and skin. However, the raw materials are not limited to these examples.

In one example of the present invention, one of the two or more kinds of individual raw materials is blood.

In one example of the present invention, one of the two or more kinds of individual raw materials is synovial tissue.

In one example of the present invention, the two or more kinds of individual raw materials are a combination of blood and synovial tissue.

The kind of the cell in the cell product according to the present invention is not particularly limited, and examples thereof include mesenchymal stem cells (synovial stem cell, bone marrow cell, or adipose stem cell), hematopoietic stem cells, embryonic stem cells, induced pluripotent cells, nerve cells, glial cells, non-glial cells, osteoblast cells, bone cells, osteoclastic cells, chondroblasts, cartilagenous cells, fibroblastic cells, keratinocytes, melanocytes, gland cells, corneal cells, retina cells, epithelial cells, platelets, thymic cells, lymphocytes, monocytes, macrophages, muscle cells, hepatic cells, renal cells, urothelial cells, myocardial cells, myoblast cells, and/or germinal cells.

In the present invention, the identification code representing the patient information is assigned to each of the two or more kinds of individual raw materials derived from the same patient. By assigning the identification code representing the patient information to the two or more kinds of individual raw materials derived from the same patient, in the manufacturing step described below, the identification code is recognized and the two or more kinds of individual raw materials are combined into one product. As a result, a cell product where the two or more kinds of raw materials derived from the same patient are combined can be manufactured.

In the present invention, the identification code representing the patient information is assigned to each of the two or more kinds of individual raw materials. Specifically, the two or more kinds of individual raw materials are accommodated in separate containers, and the identification code representing the patient information can be assigned to each of the containers. Alternatively, separate containers to which the identification code representing the patient information is previously assigned may be prepared to accommodate the two or more kinds of individual raw materials in these containers.

The form of the identification code representing the patient information is not particularly limited as long as the patient information can be identified. For example, eight or more digits (for example ten digits) of alphabets or numbers may be combined. In addition, one-dimensional code or two-dimensional code may be used. Examples of the one-dimensional code include a barcode. Examples of the two-dimensional code include a stacked two-dimensional code and a matrix two-dimensional code. Examples of the stacked two-dimensional code include PDF417. Examples of the matrix two-dimensional code include QR code (registered trademark), DataMatrix, and VeriCode. In a case where the one-dimensional code or the two-dimensional code is used, the patient information can be more simply managed by using code reading means in combination.

### <First Transport Step>

The first transport step according to the present invention is a step of transporting the two or more kinds of individual raw materials to which the identification code is assigned from a medical institution to a manufacturing site in a state where each of the raw materials is encapsulated in a double or more multiple packaging container. In the first transport step, at least one of the two or more kinds of individual raw materials is transported from the medical institution to the manufacturing site under a condition where a product of a temperature (°C) and the number of days (days) in a transport period is 170 (°C × days) or less. By transporting the raw materials from the medical institution to the manufacturing site under the above-described condition, the yield of the manufactured cells can be made sufficient.

For example, in a case where blood and synovial tissue are used as the two or more kinds of raw materials, at least one of the blood or the synovial tissue may be transported from the medical institution to the manufacturing site under a condition where a product of a temperature (°C) and the number of days (days) in a transport period is 170 (°C × days) or less. Preferably, the blood can be transported from the medical institution to the manufacturing site under a condition where a product of a temperature (°C) and the number of days (days) in a transport period is 170 (°C × days) or less.

In a case where the temperature varies in the transport period, an average temperature in the transport period can be used as the temperature in a case where the product of the temperature (°C) and the number of days in the transport period is calculated.

The condition may be set such that the product of the temperature (°C) and the number of days in the transport period is 170 (°C × days) or less and is preferably 160 (°C × days) or less, 150 (°C × days) or less, 140 (°C × days) or less, 130 (°C × days) or less, 120 (°C × days) or less, 110 (°C × days) or less, 100 (°C × days) or less, 90 (°C × days) or less, 80 (°C × days) or less, 70 (°C × days) or less, 60 (°C × days) or less, or 50 (°C × days) or less. For example, in a case where the blood is transported, by setting the product of the temperature (°C) and the number of days (days) in the transport period to be in the above-described range, the blood can be transported with high quality. As a result, by culturing synovial stem cells using the above-described blood, the synovial stem cells can be acquired with high yield. The lower limit of the product of the temperature (°C) and the number of days (days) in the transport period is not particularly limited and is generally 0.01 (°C × days) or more, 0.04 (°C × days) or more, 0.08 (°C × days) or more, or 1 (°C × days) or more.

The temperature of the first transport step is preferably 1°C to 35°C, more preferably 1°C to 30°C, still more preferably 1°C to 27°C, still more preferably 1°C to 25°C, still more preferably 5°C to 25°C, and most preferably 10°C to 20°C.

The transport period of the first transport step is preferably 1 hour or longer and 240 hours or shorter, more preferably 1 hour or longer and 192 hours or shorter, still more preferably 1 hour or longer and 160 hours or shorter, still more preferably 1 hour or longer and 150 hours or shorter, still more preferably 1 hour or longer and 144 hours or shorter, still more preferably 1 hour or longer and 130 hours or shorter, still more preferably 1 hour or longer and 120 hours or shorter, and still more preferably 1 hour or longer and 96 hours or shorter. The transport period of the first transport step may be 1 hour or longer and 72 hours or shorter, 1 hour or longer and 48 hours or shorter, 1 hour or longer and 36 hours or shorter, or 1 hour or longer and 24 hours or shorter.

For example, in a case where the raw material to be transported is blood, the period of the first transport step is preferably 1 hour or longer and 150 hours or shorter, more preferably 1 hour or longer and 144 hours or shorter, still more preferably 1 hour or longer and 130 hours or shorter, still more preferably 1 hour or longer and 120 hours or shorter, and still more preferably 1 hour or longer and 96 hours or shorter. The period of the first transport step may be 1 hour or longer and 72 hours or shorter, 1 hour or longer and 48 hours or shorter, 1 hour or longer and 36 hours or shorter, or 1 hour or longer and 24 hours or shorter.

In another example, in a case where the raw material to be transported is synovial tissue, the period of the first transport step is preferably 1 hour or longer and 130 hours or shorter, more preferably 1 hour or longer and 120 hours or shorter, and still more preferably 1 hour or longer and 96 hours or shorter. The period of the first transport step may be 1 hour or longer and 72 hours or shorter, 1 hour or longer and 48 hours or shorter, 1 hour or longer and 36 hours or shorter, or 1 hour or longer and 24 hours or shorter.

In the first transport step, it is preferable to measure and record a temperature and to trace a record of the temperature. By measuring and recording a temperature and tracing a record of the temperature, the temperature can be detected in a case where the temperature is outside of a predetermined value.

Timings at which the two or more kinds of individual raw materials are sent to the medical institution, respectively, may be the same as each other. Actually, however, it is assumed to send the two or more kinds of individual raw materials at different timings.

An interval between dates of sending at which the above-described two or more kinds of individual raw materials are sent from the medical institution, respectively, or an interval between dates of arrival at which the two or more kinds of individual raw materials arrive at the manufacturing site, respectively, is preferably 3 days to 2 weeks, more preferably 5 days to 10 days, and still more preferably 6 days to 8 days and is, for example, 7 days.

In the first transport step, the raw material to be transported is transported in the packaged state. The packaging of the raw material is preferably double packaging. For example, in a case where the raw material is blood, a blood collection bag (CELLAID (registered trademark) or a blood collection tube is used as single packaging, and a plastic bag or a plastic pack can be used as double packaging.

The raw material packaged described above is preferably transported in a state where it is accommodated in a constant-temperature transport device. The constant-temperature transport device is a device configured to maintain the raw material to be transported at a predetermined temperature, and a commercially available product (for example, TS500: manufactured by Toho Pharmaceutical Co., Ltd.) that is well-known to those skilled in the art can be used. The temperature can also be maintained using a refrigerant, an air conditioner, or the like.

The transport distance in the first transport step is not particularly limited and, for example, may be 100 m or more, 1 km or more, 10 km or more, or 100 km or more and may be 10000 km or less or 1000 km or less.

Transport means in the first transport step is not particularly limited. The raw material may be transported by a person or may be transported by various means of transportation. The means of transportation is not particularly limited and may be any one of, for example, a train, a truck, a bus, an automobile, a bicycle, a motorcycle, a ship, a helicopter, or an airplane.

### <Manufacturing Step>

The manufacturing step according to the present invention is a step including manufacturing a cell product by recognizing the identification code and combining the two or more kinds of individual raw materials into one product after the first transport step. In the present invention, by recognizing the identification code and combining the two or more kinds of individual raw materials into one product, the two or more kinds of individual raw materials derived from the same patient can be combined into one product without confusion. The manufacturing step can be performed in a manufacturing facility, a factory, or a laboratory.

In the manufacturing step, the raw material may be examined before combining the two or more kinds of individual raw materials into one product. It is preferable that the manufacturing step further includes a step of separating the two or more kinds of individual raw materials into respective members one of which is a cell, culturing the cell at a temperature of 30°C or higher, and subsequently determining whether or not the cell is suitable for a quality standard.

For example, in a case where the raw material is blood, the visual examination of the blood may be performed. The visual examination of the blood can be evaluated from the viewpoint of whether or not the state is maintained, whether a black change is recognized, or the like. In a case where it is determined that the blood is suitable for the visual examination, serum may be prepared in separation work from the blood tissue to use this serum in combination with another raw material.

For example, in a case where the raw material is synovial tissue, examination of the synovial tissue such as visual examination may be performed. The examination such as visual examination can be evaluated from the viewpoint of whether or not a change in color is recognized, whether or not the pH state is maintained, whether or not the tissue is dried as a wet state, whether or not a required amount of the tissue is included, or the like.

By mincing the synovial tissue that is suitable, immersing the minced tissue in an enzyme liquid including serum that is confirmed to be derived from the same patient using the identification code, and performing the enzyme reaction, the cell component can be produced. By immersing the produced cell component in a culture liquid including serum that is confirmed to be derived from the same patient using the identification code, putting the solution into a flask, and culturing the mixture in a sterile environment at 37°C and 5% of CO₂, the synovial tissue and the serum can be combined into one product.

In the manufacturing step, a cell product is manufactured by combining the two or more kinds of individual raw materials into one product. Preferably, a cell product can be manufactured by culturing the mixture obtained by combining the two or more kinds of individual raw materials into one product.

As culturing conditions for manufacturing the cell product, typical conditions for cell culture can be used. For example, the mixture may be cultured in a sterile environment at 25°C to 42°C (preferably 37°C) and 1 to 10% of CO₂ (preferably 5% of CO₂). The culture can be performed using a culture dish (Petri dish), a culture plate, a culture flask, a culture bag, or the like. A culture medium or conditions used for culturing the cell can be appropriately set according to the cell to be used. Specifically, for example, Eagle's minimum essential medium (EMEM), Dulbecco's modified Eagle's medium (DMEM), α-MEM, Glasgow's MEM (GMEM), IMDM, RPMI 1640, Ham's F-12, or a mixed culture medium thereof can be used, but the culture medium is not limited thereto. Further, culture mediums to which various growth factors, differentiation-inducing factors, hormones, amino acids, saccharides, salts, or the like are added may be used.

Preferably, in a case where the manufactured cell product is cleaned, a shipping inspection and shipping packaging of the final cell product can be performed.

As the shipping inspection, the number of cells (shipping standard: 1.5 × 10⁷ cells or more), the cell viability (for example, shipping standard: 70% or more), and the cell purity can be evaluated. In a case where the cell product is a product including synovium mesenchymal stem cells, As a shipping standard of the cell purity, positive for CD90 and negative for CD45 negative can be used.

Preferably, a second identification code that is coupled to the identification code assigned to the two or more kinds of individual raw materials can be assigned to the manufactured cell product. In this case, after transporting the cell product from the manufacturing site to the medical institution in the second transport step, the cell product can be allocated to the patient from which the raw materials are derived based on the above-described second identification code.

### <Second Transport Step>

The second transport step according to the present invention is a step of transporting the cell product from the manufacturing site to the medical institution after the manufacturing step.

The temperature of the second transport step is preferably 30°C or lower, more preferably 1°C to 30°C, still more preferably 2°C to 25°C, still more preferably 2°C to 22°C, still more preferably 2°C to 20°C, and still more preferably 10°C to 20°C.

The transport time in the second transport step is preferably 1 hour or longer and 90 hours or shorter, more preferably 1 hour or longer and 80 hours or shorter, still more preferably 1 hour or longer and 72 hours or shorter, still more preferably 1 hour or longer and 56 hours or shorter, still more preferably 1 hour or longer and 48 hours or shorter, and still more preferably 1 hour or longer and 32 hours or shorter.

In the second transport step, it is preferable to measure and record a temperature and to trace a record of the temperature. By measuring and recording a temperature and tracing a record of the temperature, the temperature can be maintained at a predetermined temperature.

In the second transport step, the raw material to be transported is transported in the packaged state. The packaging of the raw material is preferably double packaging. In a case where the second identification code that is coupled to the identification code assigned to the two or more kinds of individual raw materials is assigned to the cell product, it is preferable that the second identification code is assigned to a single packaging container (that is, a primary container). "Coupled" represents that the identification codes can be associated with each other irrespective of a coupling method. For example, a method of defining the association rule according to the manual or changing branch numbers of the same code for the association can also be used.

The raw material packaged described above is preferably transported in a state where it is accommodated in a constant-temperature transport device. The constant-temperature transport device is a device configured to maintain the raw material to be transported at a predetermined temperature, and a commercially available product (for example, TS500: manufactured by Toho Pharmaceutical Co., Ltd.) that is well-known to those skilled in the art can be used.

The transport distance in the second transport step is not particularly limited and, for example, may be 100 m or more, 1 km or more, 10 km or more, or 100 km or more and may be 10000 km or less or 1000 km or less.

Transport means in the second transport step is not particularly limited. The raw material may be transported by a person or may be transported by various means of transportation. The means of transportation is not particularly limited and may be any one of, for example, a train, an automobile, a ship, or an airplane.

The present invention will be described in more detail with reference to the following examples. However, the present invention is not limited to the examples.

### Examples

The following examples were performed according to the example of the system for providing a cell product in accordance with the present invention shown in Fig. 1. In the medical institution, the blood collection, the assignment of the identification code, the synovium collection, and the assignment of the identification code were performed. The blood to which the identification code was assigned and the synovium to which the identification code was assigned were transported to the manufacturing site. In the manufacturing site, serum was prepared from the blood. The serum was minced, and the minced synovium was treated with an enzyme together with the serum derived from the same patient. The synovium treated with the enzyme was cultured to manufacture a cell product. The manufactured cell product was subjected to the shipping inspection and was transported to the medical institution. In the medical institution, the cell product was administered to the patient from which the blood and the synovium were collected.

### <Example 1> Identification Step and First Transport Step regarding First Kind of Tissue (Blood Tissue) derived from Patient

As the tissue derived from the patient, blood tissue was collected from a patient in a university hospital. Specifically, using a blood separation bag (CELLAID (registered trademark)), 180 g of the blood per bag was collected from the vein of the forearm flexor portion, and 360 g of the blood in total was collected in two bags in total. A patient identification code (10 digits of a combination of alphabets and numbers) was attached to the blood tissue, the blood tissue was subjected to double packaging (the first packaging was CELLAID (registered trademark) and the second packaging was a vinyl pouch (a pack of a plastic bag), the packaging was put into a transport box (constant-temperature transport device (TS500: manufactured by Toho Pharmaceutical Co., Ltd.) of which the temperature was fixed, and the transport box was sealed. Using the transport box, the blood tissue was transported to the manufacturing site in a state where the temperature was controlled to a desired value from 1°C to 30°C. The date of sending was the same as the date of collection of the blood tissue. The transport time (which was a period where the sample was collected, was put into the constant-temperature transport device, and arrived at the manufacturing site) was 1 hour, 96 hours, 144 hours, or 192 hours.

### <Example 2> Identification Step and First Transport Step regarding Second Kind of Tissue (Synovial Tissue) derived from Patient

Seven days after the collection of the blood tissue, synovial tissue was collected from the knee in a sterile state as the second kind of tissue derived from the patient. The collected synovial tissue was encapsulated in a primary container filled with a transport medium. The patient identification code was attached to the primary container, the primary container was encapsulated in a secondary container, and the secondary container was put into an outer box. At this time, the double packaging was completed. The above-described outer box was put into a transport box (constant-temperature transport device (TS500: manufactured by Toho Pharmaceutical Co., Ltd.) of which the temperature was fixed, and the transport box was sealed. Using the transport box, the synovial tissue was transported to the manufacturing site in a state where the temperature was controlled to a desired value from 10°C to 22°C. The date of sending was the same as the date of collection of the synovial tissue. The time corresponding to the transport time was 1 hour, 120 hours, or 168 hours.

### <Example 3> Classification Step in Manufacturing Site regarding Blood

The sealing of the constant-temperature transport device transported to the manufacturing site was released, and the blood bag in the double packaging state was transported to a clean manufacturing area. In the clean manufacturing area, the packaging was released, and the visual examination of the blood was performed.

The results of the visual examination of the blood were evaluated based on the following standards. The results of the evaluation are shown in Table 1 below.
A: state maintained
B: allowable range
C: black change

The patient identification code was identified, and serum was produced in separation work from the blood tissue that was suitable. The produced serum was stored in a sterile state.

### Example 4: Classification Step in Manufacturing Site regarding Synovium

The sealing of the constant-temperature transport device transported to the manufacturing site was released, and the synovial tissue in the double packaging state was transported to a clean manufacturing area. In the clean manufacturing area, by releasing the packaging, identifying the patient identification code, mincing the synovial tissue that was suitable, immersing the minced tissue in an enzyme liquid including the serum that was prepared in Example 1 and was confirmed to be derived from the same patient using the identification code, and performing the enzyme reaction, the cell component was produced. By immersing the produced cell component in a culture liquid including the serum that was prepared in Example 1 and was confirmed to be derived from the same patient using the identification code, putting the solution into a flask, and culturing the mixture in a sterile environment at 37°C and 5% of CO₂, culture and manufacturing were performed in a sterile environment. This step is a step of combining the two kinds of raw materials derived from the patient into one product.

### Example 5: Manufacturing and Shipping Inspection of Cell Product by Culture

By culturing the mixture of the synovial tissue and the blood tissue obtained in Example 4 in a sterile environment at 37°C and 5% of CO₂ for 10 to 14 days, a cell product was manufactured. In a case where the manufactured cell product was collected from the flask and cleaned, a shipping inspection and shipping packaging of the final cell product were performed.

As the shipping inspection, the number of cells (shipping standard: 1.5 × 10⁷ cells or more), the cell viability (for example, shipping standard: 70% or more), and the cell purity (shipping standard: positive for CD90 and negative for CD45) were evaluated.

The standard yield of the number of cells (the number of synovial stem cells in a case where the cell product returned from the manufacturing site to the medical institution) was set to 1.5 × 10⁷ cells. The standard yield was the cell yield sufficient for treating one person.

The evaluation standards for the number of cells of the synovial stem cells are as follows.
A: 2 times or more of the standard yield
B: 1 time or more and less than 2 times of the standard yield
C: 0.5 times or more and less than 1 time of the standard yield
D: less than 0.5 times of the standard yield

The evaluation standards for the cell viability of the synovial tissue are as follows.
A: 80% or more and 100% or less
B: 70% or more and less than 80%
C: less than 70%

The results of the above-described evaluation are shown in Tables 1 and 2. The cell purity satisfied all the shipping standards (positive for CD90 and negative for CD45).

### [Table 1]

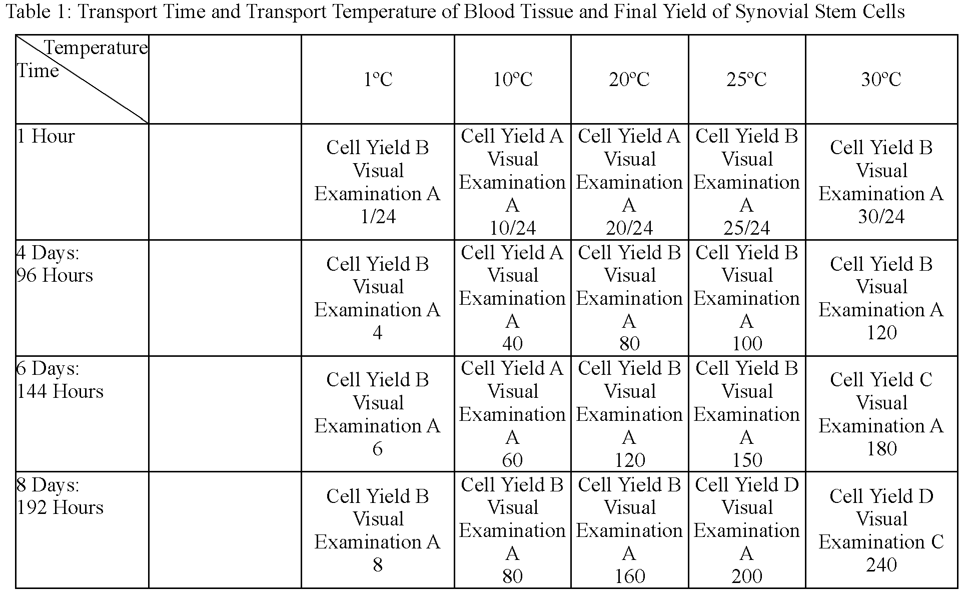

The upper stage shows the evaluation result of the cell yield.

The middle stage shows the evaluation result of the visual examination.

The lower stage shows the product of the temperature (°C) and the number of days (days) in the transport period.

### [Table 2]

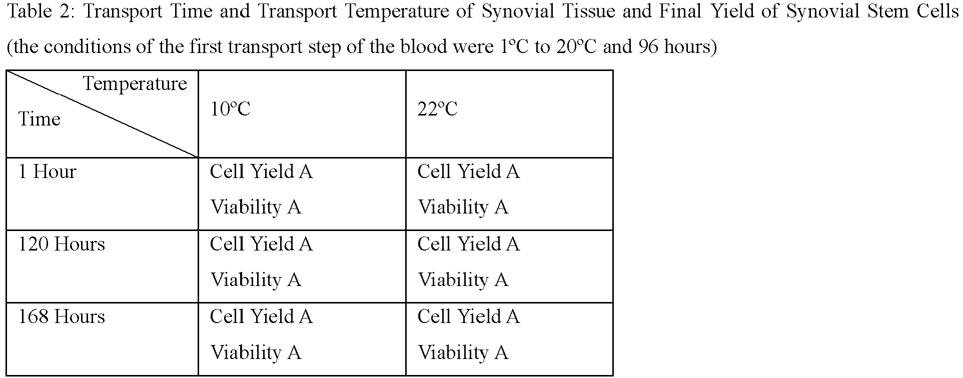

As shown above in Table 1, in a case where the blood tissue transported under a condition where the temperature × the number of days (time) was 170 or less was used, a cell product satisfying the shipping standards was able to be obtained.

### Example 6: Shipping from Manufacturing Site and Transport to Medical Institution

As shipping packaging, the synovial stem cells obtained by the culture and manufacturing of the synovial tissue and the blood tissue were suspended in the serum and were processed into a cell product form (5 × 10⁷ cells/mL) consisting of the serum and the synovial stem cells.

In one form, the serum and the synovial stem cells had the same configurations as described above and were encapsulated in a sterile state in a primary container (cryogenic tube) to which the identification code was attached, the primary container was put into a second container (NIPRO CELL GATCH (registered trademark), and the secondary container was encapsulated in an outer box and sealed (unfrozen configuration). At this time, the double packaging was completed. The above-described outer box was put into a transport box (constant-temperature transport device (TS500: manufactured by Toho Pharmaceutical Co., Ltd.) of which the temperature was fixed, the transport box was sealed, and the transport box was stored as an action corresponding to the transport. The storage temperature and time are shown in Table 3.

### [Table 3]

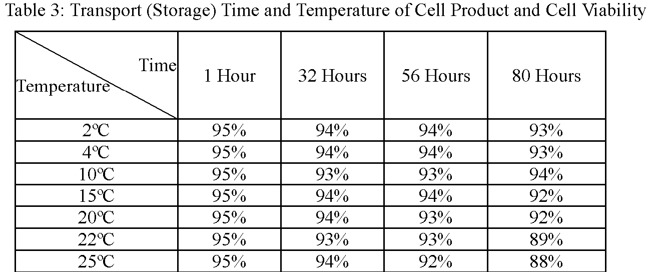

As shown in Table 3, in a case where the temperature was 2°C to 25°C and the time was 1 hour to 80 hours, the cell product was maintained in the living state (viability: 70% or more). It was found from this result that, in a case where the transport temperature is 2°C to 25°C and the transport time is 1 hour to 80 hours, the cell product can be transported from the manufacturing site and provided to the medical institution while maintaining the living state. Although the above-described temperature and time have no problem, it was found that, in a case where the temperature and time are more preferably 2°C to 20°C and 80 hours or shorter, the viability is 90% or more, which is more preferable condition for a system for providing cells.

In another form, not only the blood tissue and the synovial stem cells described above but also 5% of dimethyl sulfoxide (DMSO) were considered to be included, were encapsulated in a sterile state in a primary container to which the identification code was attached, and were frozen to -80°C or lower in a state where the primary container was put into a secondary container. At this time, the double packaging was completed. The frozen secondary container was put into an outer box and sealed, and the outer box was put into a frozen sample transport container (dry shipper) or a freezing box filled with dry ice, sealed, and stored. This action corresponds to the transport to the medical institution. At this time, in a case where the storage temperature was -60°C or lower or -80°C or lower and the transport time was 1 hour to 1 month, the cell product was able to be restored in the living state (viability: 70% or more) by being unfrozen in a warm bath at 37°C. It was found from this result that, in a case where the transport temperature is -60°C or lower or -80°C or lower and the transport time is 1 hour to 1 month, the cell product can be provided to the medical institution in a state where the cells are alive by being unfrozen in a warm bath at 37°C until arrival at the medical institution. After the cell product was transported in the frozen state at -30°C for 1 month, the cell viability after unfreezing fell below 70%. As a result, it was found that, in the frozen form, the system of the transport at -60°C or lower for 1 hour to 1 month is preferable as a system for providing cells in the living state.

### Example 7: Use of Cell Product in Medical Institution

The synovial stem cells provided in Example 6 were used for treating osteoarthrosis and for treating meniscal tear in the medical institution. Actually, it has been clarified that, by using the synovial stem cells for treating gonarthrosis, the clinical score can be improved. In addition, it was found that, by using the synovial stem cells for treating meniscal tear the clinical score is also improved. In a case where this system is used in the medical institution, the cells are administered to the patient after being recognized as cells for treatment configured with the tissues (two or more kinds) derived from the patient itself by the identification code.

As shown in Examples 1 to 7 described above, by using the system for providing cells for treatment according to the present invention, the two or more kinds of raw materials derived from the same patient can be transported from the medical institution to the manufacturing site in the living state without being mixed or confused, a cell product can be manufactured by combining the two or more kinds of raw materials derived from the same patient into one product, and the manufactured cell product can be sent to the medical institution.

## Claims

1. A system for providing a cell product, the system comprising:
an identification step of assigning an identification code representing patient information to each of two or more kinds of individual raw materials derived from the same patient;
a first transport step of transporting the two or more kinds of individual raw materials to which the identification code is assigned from a medical institution to a manufacturing site in a state where each of the raw materials is encapsulated in a double or more multiple packaging container, in which at least one of the two or more kinds of individual raw materials is transported from the medical institution to the manufacturing site under a condition where a product of a temperature (°C) and the number of days (days) in a transport period is 170 (°C × days) or less; a manufacturing step including manufacturing a cell product by recognizing the identification code and combining the two or more kinds of individual raw materials into one product after the first transport step; and
a second transport step of transporting the cell product from the manufacturing site to the medical institution after the manufacturing step.

2. The system according to claim 1,
wherein a temperature of the first transport step is 1°C to 27°C, and the transport period is 1 hour or longer and 160 hours or shorter.

3. The system according to claim 1 or 2,
wherein the manufacturing step further includes
a step of separating the two or more kinds of individual raw materials into respective members one of which is a cell, culturing the cell at a temperature of 30°C or higher, and subsequently determining whether or not the cell is suitable for a quality standard.

4. The system according to claim 1 or 2,
wherein a biological raw material other than the raw materials derived from the patient is not used.

5. The system according to claim 1 or 2,
wherein the first transport step and the second transport step include measuring and recording a temperature and tracing a record of the temperature.

6. The system according to claim 1 or 2,
wherein one of the two or more kinds of individual raw materials is blood.

7. The system according to claim 6,
wherein a period of the first transport step for the blood is 150 hours or shorter.

8. The system according to claim 1 or 2,
wherein one of the two or more kinds of individual raw materials is synovial tissue.

9. The system according to claim 8,
wherein a period of the first transport step for the synovial tissue is 130 hours or shorter.

10. The system according to claim 1 or 2,
wherein the two or more kinds of individual raw materials include blood and synovial tissue.

11. The system according to claim 1 or 2,
wherein an interval between dates of sending at which the two or more kinds of individual raw materials are sent from the medical institution, respectively, or an interval between dates of arrival at which the two or more kinds of individual raw materials arrive at the manufacturing site, respectively, is 3 days to 2 weeks.

12. The system according to claim 1 or 2,
wherein in the second transport step, a transport time is 1 hour or longer and 90 hours or shorter, and a transport temperature is 30°C or lower.

13. The system according to claim 1 or 2,
wherein a second identification code that is coupled to the identification code assigned to the two or more kinds of individual raw materials is assigned to the cell product, and the cell product is allocated to the patient from which the raw materials are derived based on the second identification code.
